Europäisches Patentamt

European Patent Office

Office européen des brevets

(19)

(11) Numéro de publication: **0 073 709**
**A1**

## DEMANDE DE BREVET EUROPEEN

(12)

(21) Numéro de dépôt: 82401550.7

(22) Date de dépôt: 18.08.82

(51) Int. Cl.³: **A 61 N 5/04**

(30) Priorité: 31.08.81 FR 8116587

(43) Date de publication de la demande: 09.03.83
Bulletin 83/10

(84) Etats contractants désignés: **DE GB NL**

(71) Demandeur: Etablissement Public dit: CENTRE
NATIONAL DE LA RECHERCHE SCIENTIFIQUE (CNRS),
15, Quai Anatole France, F-75007 Paris (FR)

(72) Inventeur: Guillon, P., 26, rue P. Brossolette,
F-87000 Limoges (FR)
Inventeur: Garault, Y., 3, rue du Masan, F-87100 Limoges
(FR)
Inventeur: Kayata, N., Cité Universitaire La Borie,
F-87000 Limoges (FR)
Inventeur: Farenc, J., 123, rue Albert-Thomas,
F-87060 Limoges (FR)

(74) Mandataire: Fruchard, Guy et al, NOVAPAT-CABINET
CHEREAU 107, boulevard Péreire, F-75017 Paris (FR)

(54) Applicateur micro-onde pour hyperthermie localisée.

(57) L'applicateur micro-onde comprend un boîtier cylindrique creux métallique (1) ayant une extrémité axiale
ouverte (3), dans laquelle est monté coaxialement un
résonateur diélectrique cylindrique (5) à forte permittivité, supporté au voisinage de l'extrémité ouverte (3) par
une bague en PTFE (6). Une sonde (11) permet d'exciter
le résonateur (5) dans les modes $TE_{01p}$ ou $EH_{11p}$ à une
fréquence relativement basse (inférieure à 1 GHz) pour
une pénétration localisé périphérique ou centrale de
l'énergie micro-onde. Application notamment au traitement des tumeurs.

ACTORUM AG

1.

La présente invention concerne les générateurs micro-ondes, et, plus particulièrement, un applicateur micro-onde portable pour hyperthermie localisée.

On sait que les micro-ondes, notamment de fréquences basses, ont des effets très marqués sur la structure des cellules animales ou végétales, ce qui a conduit à une utilisation clinique de ces micro-ondes, notamment pour le traitement de tumeurs par effet d'hyperthermie. Les dispositifs générateurs micro-ondes conventionnels sont généralement sophistiqués et encombrants et d'utilisation délicate. Par ailleurs, des résonateurs diélectriques de permittivités diverses ont été récemment developpés pour la réalisation d'oscillateurs micro-ondes stables.

La présente invention a pour objet de proposer un applicateur micro-onde à usage clinique, de dimensions réduites et de faible coût, d'utilisation particulièrement aisée et efficace, garantissant une très bonne concentration localisée de l'énergie micro-onde dans le tissu biologique à chauffer, et n'offrant que de faibles fuites par rayonnement rétrodiffusé.

La présente invention a pour autre objet de proposer un applicateur micro-onde pour hyperthermie localisée de ce type, offrant la possibilité de travailler à des fréquences basses, nonobstant des dimensions globales restreintes de l'applicateur, et assurant ainsi une meilleure pénétration de l'énergie micro-onde  dans les tissus biologiques.

Pour ce faire, selon une caractéristique de la présente invention, l'applicateur micro-onde comprend un boîtier de blindage métallique cylindrique creux définissant une chambre interne débouchant par une extrémité de travail ouverte, un résonateur diélectrique cylindrique, disposé coaxialement dans la chambre interne et ayant une première extrémité axiale s'étendant au voisinage de cette extrémité ouverte du boîtier, parallèlement à celle-ci, et des moyens commandables d'excitation du résonateur, comprenant un élément s'étendant dans la chambre au voisinage du résonateur.

Selon une autre caractéristique de la présente invention, le résonateur est réalisé en un matériau à forte permittivité et est mis en oeuvre de préférence dans les modes $TE_{01p}$ et $EH_{11p}$.

D'autres caractéristiques et avantages de la présente invention ressortiront de la description suivante d'un mode de réalisation, donné à titre illustratif mais nullement limitatif, faite en relation avec les dessins annexés, sur lesquels :

La figure 1 est une vue schématique en coupe longitudinale d'un applicateur micro-onde selon l'invention; et

Les figures 2 et 3 représentent les allures de l'amplitude du champ électrique rapporté aux dimensions transversales du résonateur   fonctionnant dans les modes $TE_{01p}$ et $EH_{11p}$, respectivement.

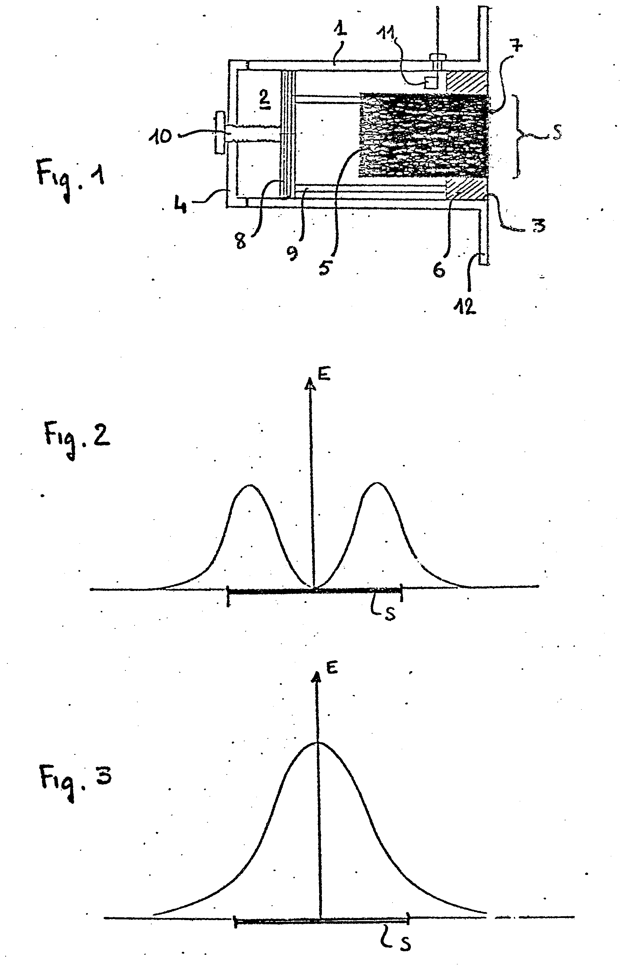

Comme représenté, sur la figure 1, l'applicateur micro-onde selon l'invention comprend un boîtier métallique de blindage 1, par exemple en aluminium, de faibles dimensions et aisément manoeuvrable manuellement, définissant une chambre interne également cylindrique 2 communiquant avec l'extérieur par une extrémité ouverte 3 du boîtier et fermée à son autre extrémité par un élément de couvercle 4 dont le réglage permet l'adaptation d'impédance de l'applicateur. Dans la chambre intérieure 2 est disposé, coaxialement à celle-ci, un résonateur diélectrique cylindrique 5, de plus petites dimensions et à forte permittivité, par exemple en céramique $S_n$, $Z_{r(1-x)}$ $T_{i O_4}$ ($x = 0,5/0,34$) développé par la société dite Thomson CSF.

Le résonateur 5 est supporté dans le boîtier 1, par l'intermédiaire d'une bague annulaire 6 en poly-tétrafluoréthylène (PTFE) au voisinage d'une de ses extrémités axiales de façon à avoir la face frontale 7 de cette extrémité axiale s'étendant au voisinage du plan de l'ouverture 3 de la chambre 2, parallèlement à ce dernier.

Dans la chambre 2 est également disposé un piston coulissant de court-circuit 8, relié à la bague support 6 par des entretoises longitudinales 9 s'étendant à distance de la périphérie du résonateur 5. Le piston 8 est relié à une vis de manoeuvre et de réglage 10 accessible de l'extérieur, du côté de la face arrière de l'applicateur, pour régler la position, dans le boîtier 1, de l'équipage du piston et du résonateur. Une sonde d'excitation 11, reliée à un dispositif oscillateur micro-ondes (non représenté ), est disposée dans l'espace annulaire entre la périphérie du résonateur cylindrique 5 et la paroi latérale adjacente du boîtier 1.

Selon une caractéristique de la présente invention, le couplage de mise en oeuvre du résonateur diélectrique est déterminé pour que celui-ci soit excité dans l'un ou l'autre des modes $TE_{01p}$ et $EH_{11p}$, dont l'allure de l'amplitude du champ électrique E est donnée sur les figures 2 et en référence par rapport à la surface d'application active S de l'applicateur. Comme on le voit, le mode $TE_{01p}$ permet de chauffer préférentiellement le pourtour d'une tumeur sur laquelle est centré l'applicateur, tandis que le mode $EH_{11p}$ concentre la

chaleur sur la partie centrale de la tumeur. On notera, à ce propos, qu'en imprimant un mouvement de balayage à l'applicateur excité sur le mode $TE_{01p}$, il est également possible de chauffer la partie centrale d'une tumeur.

Comme également représenté sur la figure 1, l'extrémité ouverte 3 du boîtier 1 est entourée par une collerette annulaire 12, avantageusement formée d'une seule pièce avec le corps du boîtier 1 et de diamètre extérieur choisi pour éliminer les fuites micro-ondes.

La fréquence de travail de l'applicateur dépend de ses dimensions, de la permittivité du résonateur diélectrique ainsi que du mode de fonctionnement choisi. Conformément à la présente invention, l'utilisation d'une forte permittivité pour le matériau électrique du résonateur permet une très bonne concentration de l'énergie micro-onde dans le tissu biologique à chauffer et de faibles fuites par rayonnement rétrodiffusé. De plus, avec cet agencement, il est possible de travailler à des fréquences basses (inférieures à 1 GHz) en conservant des dimensions d'applicateurs compatibles avec la taille des tumeurs à traiter. La possibilité de travailler à des fréquences basses permet en outre une meilleure pénétration de l'énergie micro-onde dans les tissus biologiques.

On soulignera par ailleurs, ici, la possibilité de créer, dans des tissus biologiques, des zones de champs formés par l'interférence du rayonnement de deux applicateurs du type de celui représenté sur la figure 1.

Quoique la présente invention ait été décrite en relation avec un mode de réalisation particulier, elle ne s'en trouve pas limitée, mais est au contraire susceptible de modifications et de variantes qui apparaîtront à l'homme de l'art.

REVENDICATIONS

1 - Applicateur micro-onde pour hyperthermie localisée, caractérisé en ce qu'il comprend un boîtier de blindage métallique cylindrique creux (1) définissant une chambre interne (2) débouchant axialement par une extrémité ouverte (3) du boîtier, un résonateur diélectrique cylindrique (5) disposé coaxialement dans la chambre intérieure (2) et ayant une première extrémité axiale (7) s'étendant au voisinage de l'extrémité ouverte (3) du boîtier (1), parallèlement à celle-ci, et des moyens commandables d'excitation du résonateur (5) comprenant un élément (11) s'étendant dans la chambre interne (2) au voisinage du résonateur (5).

2 - Applicateur selon la revendication 1, caractérisé en ce que le résonateur (5) est supporté, au niveau de sa première extrémité (7), dans la chambre interne (2) au moyen d'une bague (6) en matériau plastique.

3 - Applicateur selon la revendication 1 ou la revendication 2, caractérisé en ce qu'il comprend un piston de court-circuit (8) monté à coulissement commandable dans la chambre interne (2), à distance axiale de la seconde extrémité axiale du résonateur (5).

4 - Applicateur selon la revendication 3, caractérisé en ce que le piston (8) est relié (9) à la bague support (6).

5 - Applicateur selon la revendication 3 ou la revendication 4, caractérisé en ce qu'il comprend un moyen d'actionnement (10) accessible de l'extérieur du boîtier pour déplacer sélectivement le piston (8).

6 - Applicateur selon l'une des revendications 2 à 5, caractérisé en ce que la bague support (6) est en polytétrafluoréthylène.

7 - Applicateur selon l'une des revendications précédentes, caractérisé en ce que le résonateur est excité dans le mode $TE_{01p}$.

8 - Applicateur selon l'une des revendications 1 à 6, caractérisé en ce que le résonateur (5) est excité dans le mode EH$_{11p}$.

9 - Applicateur selon l'une des revendications précédentes, caractérisé en ce que le boîtier (1) comprend une collerette annulaire (12) s'étendant autour de son extrémité ouverte (3).

10 - Applicateur selon l'une des revendications précédentes, caractérisé en ce que le résonateur (5) est en céramique à forte permittivité.

0073709

1/1

Fig. 1

Fig. 2

Fig. 3

Office européen
des brevets

**RAPPORT DE RECHERCHE EUROPEENNE**

EP 82 40 1550

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 N 5/04 |
| A | DE-B-1 006 086 (SCHLIEBS) *Colonne 3, lignes 10-23; figures 1,2* | 1 | |
| | --- | | |
| A | US-A-2 407 690 (SOUTHWORTH) *Colonne 4, lignes 61-73; colonne 5, lignes 44-62; figures 1,2* | 1,3,10 | |
| | --- | | |
| A | GB-A- 862 646 (ZEISS) *Page 2, lignes 27-33; figures* | 3 | |
| | --- | | |
| A | FR-A-2 421 628 (CGR-MeV) *Page 9, lignes 30-32* | 6 | |
| | --- | | |
| A | US-A-3 845 267 (FITZMAYER) *Colonne 3, lignes 28-49; figure 2* | 7 | DOMAINES TECHNIQUES RECHERCHES (Int. Cl. ³) |
| | --- | | |
| A | US-A-3 527 227 (FRITZ) *Colonne 2, lignes 41-47; figure 1* | 9 | A 61 N H 05 B |
| | --- | | |
| A | WO-A-8 001 462 (TURNER) | 1 | |
| | ----- | | |

Le présent rapport de recherche a été établi pour toutes les revendications

| Lieu de la recherche LA HAYE | Date d'achèvement de la recherche 06-12-1982 | Examinateur BIJN E.A. |
|---|---|---|